# EUROPÄISCHE PATENTSCHRIFT

(11) **EP 2 593 057 B1**
(45) Veröffentlichungstag und Bekanntmachung des Hinweises auf die Patenterteilung: **03.09.2014**
(21) Anmeldenummer: 11745508.9
(22) Anmeldetag: 14.07.2011
(51) Int. Cl.: A61F 9/008

(54) **STEUERDATENERZEUGUNG FÜR DIE AUGENCHIRURGISCHE FEHLSICHTIGKEITSBEHANDLUNG**
CONTROL DATA GENERATION FOR THE EYE-SURGICAL TREATMENT OF DEFECTIVE VISION
GÉNÉRATION DE DONNÉES DE COMMANDE POUR LE TRAITEMENT CHIRURGICAL OPHTALMIQUE DE L'AMÉTROPIE

(30) Priorität: 14.07.2010 DE 102010031348
(43) Veröffentlichungstag der Anmeldung: 22.05.2013
(73) Patentinhaber: Carl Zeiss Meditec AG, 07745 Jena (DE)
(72) Erfinder: BISCHOFF, Mark, 07749 Jena (DE); STOBRAWA, Gregor, 07743 Jena (DE); BERGT, Michael, 99425 Weimar (DE)
(74) Vertreter: Geyer, Fehners & Partner
(86) Internationale Anmeldenummer: PCT/EP2011/062067
(87) Internationale Veröffentlichungsnummer: WO 2012/007552

(56) Entgegenhaltungen:
- EP-A1- 1 897 520
- WO-A1-2005/011547
- DE-A1-102008 017 772

## Beschreibung

Die Erfindung bezieht sich auf eine Vorrichtung sowie ein Verfahren zur Erzeugung von Steuerdaten, die ausgebildet sind zur Ansteuerung einer Laserstrahlung abgebenden Lasereinrichtung zur operativen Fehlsichtigkeitskorrektur eines Auges, wobei eine Schnittfläche vorgegeben ist, die gekrümmt ist, einen Scheitelpunkt und einen Rand hat und zur Fehlsichtigkeitskorrektur im Auge zu erzeugen ist, für die Steuerdaten eine oder mehrere Bahnkurve(n) definiert wird/werden, entlang der ein Fokus der Laserstrahlung zu verstellen ist, die Bahnkurve(n) so gewählt wird/werden, daß sie in der Schnittfläche oder innerhalb eines Toleranzbereiches um die Schnittfläche liegt/liegen.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzfläche mindert.

Zur Schnitterzeugung wird eine Serie optischer Durchbrüche an vorbestimmten Stellen so erzeugt, daß dadurch die Schnittfläche ausgebildet wird. Beim Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Man sieht deshalb in der LASIK-Methode für den Ablationslaser ein sogenanntes shot file vor, das für verschiedene Punkte auf der Augenhornhaut festlegt, wie oft der Laserstrahl auf definierte Punkte auf der Hornhaut gerichtet werden soll und mit welcher Energie. Die Volumenentfernung wurde dabei heuristisch ermittelt, nicht zuletzt da sie sehr von der Ablationswirkung des Laserstrahls, mithin von der Wellenlänge, Fluence etc. der eingesetzten Strahlung abhängt. Auch spielt der Zustand der Augenhornhaut eine Rolle; hier ist insbesondere der Feuchtigkeitsgehalt der Augenhornhaut zu nennen. Die WO 96/11655 schildert eine Vorrichtung und ein Verfahren für die LASIK-Methode. Dabei wird insbesondere eine Formel angegeben, die aus dem vor-operativen Hornhautkrümmungsradius und der gewünschten Dioptrienkorrektur den zu erreichenden Hornhautkrümmungsradius berechnet. Eine ähnliche Berechnung ist in der EP 1153584 A1 beschrieben - ebenfalls für die Hornhautablation mittels LASIK.

Ein weiteres laserbasiertes, augenchirurgisches Verfahren liegt darin, das zu entfernende Hornhautvolumen nicht zu verdampfen, sondern durch einen Laserschnitt zu isolieren. Das Volumen wird also nicht mehr abladiert, sondern in der Hornhaut durch eine dreidimensionale Schnittfläche isoliert und somit entnehmbar gemacht. Für solche Verfahren sind Erfahrungswerte, die zum Schleifen der Hornhaut mittels Ablationslaserstrahlung entwickelt wurden, nicht brauchbar. Statt dessen werden Steuerdaten für den Betrieb des Lasers zur Isolation des zu entfernenden Hornhautvolumens benötigt. In der US 6110166 und der US 7131968 ist ein solches augenchirurgisches Verfahren beschrieben. Dabei sind in US 6110166 verschiedene Volumenformen gezeigt, und es ist erwähnt, daß der Fachmann das passende Volumen auswählen kann.

Die DE 102006053118 A1 schildert die Erzeugung von Steuerdaten für die volumenisolierende Fehlsichtigkeitskorrektur.

Aus der DE 102006053120 A1 und der DE 102006053119 A1 der Carl Zeiss Meditec AG ist es bekannt, bei der Erzeugung solcher Steuerdaten von Fehlsichtigkeitsdaten auszugehen, welche die Brechkraft einer für die Fehlsichtigkeitskorrektur tauglichen Brille angeben. Auch ist es aus dieser Druckschrift, die somit ein gattungsgemäßes Verfahren und eine gattungsgemäße Vorrichtung schildert, bekannt, Daten zu verwenden, die auch eine Astigmatismuskorrektur oder Korrekturen höherer Aberrationsordnungen bewirken.

Für die volumenisolierende Fehlsichtigkeitskorrektur ist die Präzision, mit der die erforderlichen Schnittflächen erzeugt werden, von großer Bedeutung. Anders als beim Laserkeratom hat die Lage der Schnittflächen direkte Auswirkung auf die Qualität der optischen Korrektur. Bei der herkömmlichen LASIK-Methode ist dagegen ausschließlich die Präzision, mit der die Laserablation betrieben wird, für die Güte der optischen Korrektur wichtig. Dies erkennt man schon daraus, daß die Erzeugung der Hornhautlamelle in einer großen Vielzahl von Operationen mit einem vergleichsweise grob arbeitenden, mechanischen Messer praktiziert wird bzw. wurde.

Da für die Präzision der Schnittflächenerzeugung die exakte Positionierung des Auges wichtig ist, schlägt der Stand der Technik, beispielsweise die WO 2005/011547 A1 vor, daß bei laserchirurgischen Vorrichtungen ein Kontaktglas verwendet werden kann, an das die Hornhaut angepreßt wird. Dieses Kontaktglas dient dazu, das Auge zu fixieren.

Für die Präzision der Schnittflächen ist aber nicht nur die präzise Lage des Auges wichtig, auch die Form der Hornhaut muß bekannt sein. Da diese von Patient zu Patient innerhalb gewisser Bereiche variiert, dient das Kontaktglas auch dazu, der Hornhautvorderfläche eine feste, definierte Form zu geben. Beim Anpressen der Hornhautvorderseite an das Kontaktglas kommt es folglich zu einer Deformation der Hornhaut, die je nach Abweichung der Kontaktglaskrümmung von der natürlichen Hornhautkrümmung des jeweiligen Patienten unterschiedlich groß ist.

Ist die Lage der Schnittflächen für die optische Korrektur von Bedeutung, d. h. wird nicht nur eine Lamelle isoliert und das zu entfernende Volumen durch Ablation entfernt, ist die Deformation der Hornhaut bei der Bestimmung der Zielkoordinaten für die Schnittflächenerzeugung wesentlich. Es ist deshalb im Stand der Technik bekannt, die Deformation dadurch zu berücksichtigen, daß die vorher ermittelten Zielpunkte einer Koordinatentransformation unterzogen werden. In der genannten WO-Veröffentlichung wird diese Transformation als "Anpreßtransformation" bezeichnet und es werden Transformationsgleichungen für eine Kombination aus sphärischem Kontaktglas und sphärischer Hornhautvorderfläche gegeben. Die DE 102008017293 A1 der Carl Zeiss Meditec AG ergänzt diese Transformationsgleichungen, so daß auch bei andersartigen Kontaktglas- und Hornhautkrümmungen die Koordinatentransformation vorgenommen werden kann.

Die Erfindung betrifft also das Konzept, eine Korrektur der optischen Abbildungsfehler des menschlichen Auges dadurch durchzuführen, daß mittels Laserstrahlung in der Augenhornhaut eine Separierung eines Gewebevolumens erreicht wird, welches dann aus der Hornhaut entfernt wird. Dadurch wird eine gezielte Änderung der Brechkraft der Augenhornhaut erreicht. Diese Änderung erfolgt lokal, d. h. in dem Bereich der Hornhaut, aus dem das Gewebevolumen entnommen wird. Üblicherweise orientiert man sich dabei an der Pupille des Auges.

Die Entnahme des separierten Volumens verändert die Geometrie, nämlich die Krümmung der Hornhautoberfläche. Damit eine gewünschte Fehlsichtigkeitskorrektur erreicht wird, muß deshalb das separierte und zu entnehmende Volumen hinsichtlich seiner Form spezielle Eigenschaften aufweisen.

Wie bereits erläutert ist für die augenchirurgische Fehlsichtigkeitskorrektur die Krümmung, welche die Vorderfläche der Augenhornhaut nach der Korrektur hat, ausschlaggebend für den Korrektureffekt. Es ist deshalb bei der Volumen isolierenden Fehlsichtigkeitskorrektur erforderlich, diese Flächenform zu berücksichtigen. Das Hauptaugenmerk lag dabei in der Vergangenheit, wie bereits erwähnt, in einer sphärischen oder zylindrischen Korrektur. Da die entsprechende Vorderflächenänderung am unverformten Auge stattfinden muß, war es von besonderem Vorteil, daß diese Korrekturflächen analytisch beschrieben werden konnten. In diesem Fall war auch die Transformation, welche durch das Anpressen des Kontaktglases auszuführen ist, analytisch beschreibbar.

Der Vorteil der analytischen Beschreibbarkeit zeigt sich besonders dann, wenn die entsprechenden Zielpunkte für die Laserstrahlung festgelegt und eine Bahnkurve definiert werden, welche die Zielpunkte verbindet und entlang welcher der Fokus einer Behandlungslinsenstrahlung verstellt werden muß.

Es war deshalb bislang relativ einfach möglich, die Bahnkurven so zu bestimmen, daß eine schnelle Abarbeitung der einzelnen Zielpunkte erreicht werden konnte. Im Stand der Technik ist es dazu bekannt, den Laserstrahl längs Höhenlinien der Schnittfläche zu führen oder längs einer Spirale, die sich an Höhenlinien der Schnittfläche orientiert. Die erforderliche Zerlegung der Schnittfläche in Höhenlinien oder einer Höhenlinienspirale ließ sich aufgrund der einfachen analytischen Beschreibbarkeit auch der transformierten Schnittfläche vergleichsweise einfach bewerkstelligen.

Möchte man jedoch Schnittflächenkorrekturen vornehmen, die nicht mehr analytisch beschreibbar sind oder zumindest im transformierten Zustand, d.h. in dem Zustand des Auges, in dem die Schnittfläche erzeugt wird, nicht mehr analytisch beschreibbar sind, ist es äußerst schwierig, eine geeignete Wahl der Zielpunkte bzw. der Bahnkurve, entlang der die Zielpunkte aufgereiht sind, auszuführen.

Der Erfindung liegt deshalb die Aufgabe zugrunde ein gattungsgemäßes Verfahren bzw. eine gattungsgemäße Vorrichtung dahingehend weiterzubilden, daß für eine beliebige, insbesondere nicht analytisch darstellbare Schnittfläche, welche im zum Zeitpunkt der Operation vorliegenden Auge (gegebenenfalls verformt durch ein Kontaktglas) erzeugt werden soll, eine entsprechende Bahnkurve bzw. entsprechende Bahnkurven anzugeben.

Diese Aufgabe wird erfindungsgemäß mit einem Verfahren der eingangs genannten Art gelöst, bei dem eine Bezugsebene, welche in einem vorgegebenem Winkel, vorzugsweise senkrecht, zu einer Einfallsrichtung der Laserstrahlung ist, festgelegt und für diese verschiedene Verschiebestellungen vom Scheitelpunkt zum Rand der Schnittfläche festgelegt werden, für jede Verschiebestellung mehrere Achsen oder Halbachsen festgelegt werden, die in der Bezugsebene liegen, zueinander nicht parallel sind und sich, gesehen in einer Projektion längs der Einfallsrichtung innerhalb des Randes der Schnittfläche, im Falle von Achsen kreuzen oder im Falle von Halbachsen zusammenlaufen, für jede Verschiebestellung der Bezugsebene Schnittpunkte der Achsen bzw. Halbachsen mit der Schnittfläche ermittelt werden und die Bahnkurve(n) festgelegt wird/werden, indem die Schnittpunkte durch eine Interpolation zu in Projektion längs der Einfallsrichtung konzentrischen, geschlossenen Kurven oder in Form einer Spirale verbunden werden.

Die Aufgabe wird weiter mit einer Vorrichtung zur Erzeugung von Steuerdaten gelöst, die ausgebildet sind zur Ansteuerung einer Laserstrahlung abgebenden Lasereinrichtung zur operativen Fehlsichtigkeitskorrektur eines Auges, wobei die Vorrichtung aufweist: eine Schnittstelle zum Empfang von Schnittstellendaten, die eine Schnittstelle vorgeben, die gekrümmt ist, einen Scheitelpunkt und einen Rand hat und zur Fehlsichtigkeitskorrektur im Auge zu erzeugen ist, eine Recheneinrichtung, die eine oder mehrere Bahnkurve(n) definiert, entlang der ein Fokus der Laserstrahlung zu verstellen ist, wobei die Recheneinrichtung die Bahnkurve(n) gemäß dem obigen Verfahren.

Unter dem Begriff konzentrische, geschlossene Kurven werden mehrere Kurven verstanden, die jeweils geschlossen sind, sich nicht schneiden und in einem gemeinsamen Punkt zusammengezogen werden können. Die Bahnkurve(n) liegt/liegen somit in der Schnittfläche oder innerhalb eines Toleranzbereiches um die Schnittfläche.

Die Erfindung stellt ein Verfahren zur Zerlegung einer Schnittfläche in Höhenlinien oder in eine an Höhenlinien orientierte Spirale bereit, bei der eine Parameterdarstellung der Schnittfläche, die entweder mathematisch sehr kompliziert sein kann, oder gegebenenfalls auch analytisch überhaupt nicht mehr möglich ist, nicht erforderlich ist. Die Schnittfläche wird mit Hilfe der nicht zueinander parallel liegenden und sich in einer Bezugsebene kreuzenden Achsen bzw. dort zusammenlaufenden Halbachsen in diskrete Stützstellen (oder auch Funktionswerte) zerlegt. Diese Achsen (bzw. Halbachsen werden nun senkrecht zur Bezugsebene verschoben, was gleichbedeutend damit ist, daß die Bezugsebene verschoben wird. Jede Verschiebung entspricht einer Verschiebestellung. Möchte man eine Spirale erzeugen, sind die diskreten Funktionswerte durch die Verschiebestellungen beispielsweise um einen konstanten Verschiebewert beabstandet.

Die Erfindung kann mit sich kreuzenden Achsen arbeiten. Alternativ kann auch mit Halbachsen gearbeitet werden, die in einem Punkt zusammenlaufen. Es sei darauf hingewiesen, daß die Arbeit mit Halbachsen es auch erlaubt, eine nicht symmetrische Achsenanordnung zu wählen bzw. mit einer ungeraden Anzahl von Halbachsen zu arbeiten, beispielsweise mit Halbachsen die vom Zentrum auf den Stern eines Fünfecks laufen o.ä. Soweit nachfolgend lediglich von Achsen gesprochen wird, soll das die Verwendung von Halbachsen nicht ausschließen.

Die Bahnkurven (im Falle konzentrischer geschlossener Kurven) oder die entlang der Höhenlinien verlaufenden Spirale wird dann durch stückweise radiale Interpolation der Stützstellen erhalten. Dabei ist es zweckmäßig, daß die Interpolation stückweise so zwischen jeweils zwei Stützstellen, d.h. Schnittpunkten erfolgt, daß die Übergänge zwischen zwei aufeinanderfolgenden Interpolationsstücken glatt sind. Unter glatten Übergängen werden im Sinne dieser Beschreibung stetig differenzierbare Übergänge verstanden.

Erfindungsgemäß kann anders als aus der WO2005011547 eine Schnittfläche unabhängig von deren analytischer Darstellung in Höhenlinien oder eine an Höhenlinien orientierte Spirale zerlegt werden. Insbesondere unter dem Gesichtspunkt der Scangeschwindigkeit ist dies vorteilhaft, denn für jede Fläche *F:R²→R³* im Raum existiert jeweils eine Schnittlinie *F:R→R²* mit einer Ebene senkrecht zur langsamsten Scandimension, die dann im zweidimensionalen Unterrraum der verbleibenden schnellen Scandimensionen liegt. Variiert man die Lage der Ebene senkrecht zur langsamsten Scandimension, so ergeben sich verschiedene Schnittlinien, die man im Unterraum der schnellen Scandimensionen abfahren (scannen) muß. Diese Linien sind dann Höhenlinien bezüglich der langsamsten Scandimension, wodurch die Anforderungen an die Verstellgeschwindigkeit erheblich abgesenkt werden. Es ist für den Fachmann klar, daß eine Bewegung entlang benachbarter Höhenlinien im Prinzip in einer spiralförmigen Bahnkurve erfolgen kann. Die Laufrichtung der Spirale spielt dabei keine Rolle, rechts- oder linksdrehend, Ein- oder Auswärtsbewegung sind für den Fachmann vom Konzept erfaßt.

Bei den bekannten Scaneinrichtungen ist die Verstellgeschwindigkeit entlang der optischen Achse (z-Achse genannt) am geringsten. Daher erfolgt eine Zerlegung in Höhenlinien bevorzugt in diese Raumdimension. Abstrakter formuliert, sucht man eine parametrische Darstellung der Schnittfläche F, die bei Variation des einen Parameters die z-Ablage der Punkte auf der Fläche nicht ändert.

Die bereits erwähnte Anpreßtransformation hat eine problematische Eigenschaft. Wendet man sie auf eine Höhenlinie an, so ist im Allgemeinen die transformierte Kurve keine Höhenlinie mehr. Es besteht also die Aufgabe, ein Verfahren zur Berechnung von Schnittlinien im angepreßten Zustand (Kontaktglassystem) zu finden, die dort Höhenlinien sind. Hierbei handelt es sich um ein kompliziertes mathematisches Problem, dessen Lösung im Allgemeinen durchaus erheblichen Rechenaufwand verursachen kann, denn es müssen Höhenlinien bzw. eine daran orientierte Spirale auf einer i.d.R. analytisch nicht mehr darstellbaren Fläche gefunden werden.

Dies ist vor allem dann sehr aufwendig, wenn Schnittflächen die mehr als 2-zählige Winkelsymmetrien (bezüglich Rotation um die optische Achse) aufweisen, wie sie insbesondere bei einer Korrektur von Fehlsichtigkeiten höherer Ordnung auftreten.

Für die Erzeugung der geschlossenen Bahnkurven bzw. der anhand Höhenlinien orientierten Spirale sind Zylinderkoordinaten vorteilhaft. Es ist deshalb bevorzugt, daß die Interpolation in Zylinderkoordinaten erfolgt, wobei die Achsen bzw. Halbachsen so gewählt werden, daß eine Zylinderachse der Zylinderkoordinaten in jeder Verschiebestellung nahe eines Kreuzungspunktes der Achsen bzw. gemeinsamen Punktes der Halbachsen liegt oder mit diesem zusammenfällt. Die Zylinderachse steht senkrecht zur Bezugsebene. Besonders rechensparend ist das Verfahren dann, wenn sich alle Achsen auf der Zylinderachse kreuzen. Eine Symmetrie der Schnittfläche kann dabei vorteilhaft ausgenutzt werden, wenn weiter die Zylinderachse vom Scheitelpunkt zum Zentrum des Randes der Schnittfläche verläuft. Im Hinblick auf den Einsatz an der Lasereinrichtung ist es weiter zu bevorzugen, wenn die Zylinderachse mit der z-Achse der Scaneinrichtung zusammenfällt.

Die Anzahl und Winkellagen *ϕₖ* der für die Schnittpunktgewinnung benötigten charakteristischen Achsen ist prinzipiell unabhängig von der jeweiligen Struktur der zu zerlegenden Schnittfläche F. Legt man deren Anzahl fest, und verteilt beispielesweise die Winkellagen *ϕₖ* gleichmäßig, so ist auch die Komplexität der dadurch genäherten Fläche begrenzt (Samplingtheorem). Man muß also die Anzahl der Achsen groß genug wählen, um eine hinreichend gute Annäherung zwischen der auf diese Weise bestimmten Bahnkurve(n) *H(ϕ)* und der zu zerlegenden Schnittfläche F zu erreichen. Die Anzahl der Achsen kann beispielsweise so groß gewählt werden, daß der Abstand der Bahnkurve(n) H von der Schnittfläche F kleiner ist als 10um, insbesondere kleiner ist als 1 um, insbesondere kleiner ist als 0.1 µm.

Der Rechenaufwand für die Bahnkurvenberechnung steigt aber zunehmend mit der Anzahl der Achsen, und nicht immer ist die Komplexität der zu zerlegenden Fläche so hoch, das man eine fest vorgegebene Anzahl von Achsen benötigt. Vorteilhafterweise wird daher die Anzahl der Achsen minimiert und die Auswahl und Lage so gewählt, daß sie der Komplexität der Schnittfläche angepaßt sind. Das führt zu einer ebenso guten Anpassung wie die Verwendung von weiteren Achsen.

Die für eine bestimmte Schnittfläche F notwendige Anzahl von Achsen ermittelt man zweckmäßigerweise aus der Winkelsymmetrie der Fläche. Es ist deshalb bevorzugt, eine die Winkelsymmetrie der Schnittfläche beschreibende Symmetriezahl zu ermitteln und die Zahl der Achsen gleich der Symmetriezahl zu wählen. Dabei ist zweckmäßigerweise die Symmetriezahl gleich der Zahl der Symmetrieachsen, die für die Schnittfläche angegeben werden können. Bei einer elliptischen Schnittfläche entstehen beispielsweise zwei Symmetrieachsen, so daß man zwei Achsen für die Bahnkurvenbestimmung verwenden wird.

Hinsichtlich der Symmetriezahl ist es bevorzugt, daß zur Bestimmung der Symmetriezahl zuerst eine Höhenfunktion z(r, ϕ) der Schnittfläche auf einem Kreis mit dem Radius R bestimmt wird und dann diese Höhenfunktion in eine Funktionsreihe entwickelt wird, wobei die Symmetriezahl bei Achsen als die maximale Ordnung der Funktionsreihe bei Halbachsen als das Doppelte der maximalen Ordnung festgelegt wird, die nötig ist, um eine Abweichung zwischen Entwicklung und Höhenfunktion unter einen zuvor gewählten Schwellwert zu drücken.

Man bestimmt also zunächst eine in ϕ periodische radiale Höhenfunktion z(r, ϕ) der Schnittfläche F auf einem Kreis mit dem Radius R. Dann legt man eine Signifikanzschwelle fest und entwickelt die Höhenfunktion in eine Zernike- oder Fourierreihe bis zu der Ordnung, bei der die Abweichung der Reihe von der Höhenfunktion kleiner als die Signifikanzschwelle ist. Die Signifikanzschwelle ist dabei vorzugsweise kleiner als 10µm, insbesondere kleiner als 1 µm, insbesondere kleiner als 0.1 µm. Die Anzahl notweniger Achsen ist dann gleich der maximal notwendigen Ordnung der Reihe. Die Winkellagen der Achsen können dann gleichmäßig verteilt sein.

Diese Analyse wird vorzugsweise für alle Radien R <= Rₘₐₓ durchgeführt, wobei Rₘₐₓ die maximale spätere Ausdehnung der Schnittlinie z. B. des Randes der Schnittfläche ist. Die daraus resultierend maximale Achsenanzahl wird verwendet.

Eine weitere Möglichkeit zur Bestimmung der Anzahl und Lage charakteristischer Achsen ist der Anzahl und Lage der lokalen Extrema der periodischen radialen Höhenfunktion. Man legt dann die Achsen (bzw. Halbachsen) stets auf die Winkellagen aller Minima und Maxima.

Es ist also ebenfalls möglich, aus der Höhenfunktion die Winkellagen lokaler Extrema dieser Höhenfunktion zu ermitteln und die Achsen auf die Winkellagen der lokalen Extrema zu legen. Eine Winkelgleichverteilung muß dann nicht zwingend gegeben sein. Das oben beschriebene Vorgehen läßt sich gleichermaßen für die Erzeugung geschlossener konzentrischer Bahnkurven, die Höhenlinien der Schnittfläche zumindest approximieren, als auch zur Erzeugung einer Spirale, die an solchen Höhenlinien oder Bahnkurven orientiert ist, verwenden. Der Unterschied liegt im wesentlichen darin, ob aufeinanderfolgend interpolierte Schnittpunkte in derselben Verschiebestellung der Bezugsebene, z.B. in der selben z-Ebene liegen, oder nicht. Liegen die aufeinanderfolgend verbundenen Schnittpunkte in derselben Ebene, erhält man eine Bahnkurve. Werden Schnittpunkte verbunden, die in unterschiedlichen Verschiebestellungen (oder z-Lagen) angeordnet sind, erhält man eine Spirale. Beiden Vorgehensweisen ist jedoch gleich, daß immer Schnittpunkte verbunden werden, die aus winkelbenachbarten Achsen erhalten wurden.

Eine präzise Winkelangabe der Achsen ist dann besonders einfach möglich, wenn alle Achsen sich in einem Kreuzungspunkt schneiden bzw. alle Halbachsen in einem einzigen Punkt enden. Eine solche Wahl der Achsen ist deshalb bevorzugt. Das erfindungsgemäße Verfahren ist jedoch darauf nicht beschränkt, vielmehr können auch mehrere Schnittpunkte vorhanden sein. Der Bezugspunkt für die Winkelangabe bzw. der Durchstoßpunkt der Zylinderachse durch die Bezugsebene ist dann zweckmäßigerweise so gelegt (bzw. die Achsen sind bezüglich der Zylinderachse so gelegt), daß dieser Bezugspunkt nahe einem gemeinsamen Punkt der Achsen (Halbachsen) liegt. Die eigentlich auf den jeweiligen gemeinsamen Punkt zu beziehende Ortsangabe dann auf den genannten Bezugspunkt zu referenzieren, führt in aller Regel zu einem tolerierbaren Fehler. Im Ergebnis müssen sich somit nicht alle Achsen in einem Punkt schneiden, auch wenn dies aus verringertem Rechenaufwand zu bevorzugen ist.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Das erfindungsgemäße Verfahren zum Erzeugen der Steuerdaten kann ohne menschliche Mitwirkung ausgeführt werden. Insbesondere können sie von einem Computer durchgeführt werden, der unter Steuerung eines erfindungsgemäßen Programms das erfindungsgemäße Verfahren ausführt und aus entsprechenden Vorgaben die Steuerdaten für die Lasereinrichtung ermittelt. Insbesondere ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Soweit in dieser Beschreibung ein Verfahren bzw. einzelne Schritte eines Verfahrens zur Ermittlung von Steuerdaten zur optischen Fehlsichtigkeitskorrektur beschrieben wird, kann das Verfahren bzw. können einzelne Schritte des Verfahrens durch die entsprechend ausgestaltete Vorrichtung ausgeführt werden. Analoges gilt für die Erläuterung der Betriebsweise einer Vorrichtung, die Verfahrensschritte ausführt. Insoweit sind Vorrichtungs- und Verfahrensmerkmale dieser Beschreibung äquivalent. Insbesondere ist es möglich, das Verfahren mit einem Computer zu realisieren, auf dem ein entsprechendes erfindungsgemäßes Programm ausgeführt wird.

Die Erfindung wird nachfolgend unter Bezugnahme auf die Zeichnungen beispielshalber noch näher erläutert. In den Zeichnungen zeigt:
- Fig. 1: eine Schemadarstellung einer Behandlungsvorrichtung bzw. eines Behandlungsgerätes zur Fehlsichtigkeitskorrektur,
- Fig. 2: eine Schemadarstellung hinsichtlich des Aufbaus des Behandlungsgerätes der Fig. 1,
- Fig. 3: eine Prinzipdarstellung zur Einbringung gepulster Laserstrahlung in das Auge bei der Fehlsichtigkeitskorrektur mit dem Behandlungsgerät der Fig. 1,
- Fig. 4: eine schematische Schnittdarstellung durch die Augenhornhaut zur Veranschaulichung der Volumenentnahme,
- Fig. 5: eine schematische Darstellung einer Schnittfläche mit vier Achsen zur Erzeugung einer Bahnkurve,
- Fig. 6: die zugehörige erzeugte Bahnkurve in Form einer an Höhenlinien orientierten Spirale,
- Fig. 7: eine beispielhafte Schnittfläche mit dreizähliger Symmetrie,
- Fig. 8: eine Veranschaulichung zur Funktionsreihenentwicklung der Fläche der Fig. 7 in einer Höhenfunktion,
- Fig. 9: die Schnittfläche der Fig. 7 mit den ermittelten Achsen und
- Fig. 10: eine Darstellung der Schnittfläche der Fig. 7 mit ermittelten Schnittpunkten und einer an Höhenlinien orientierten Spirale als Bahnkurve.

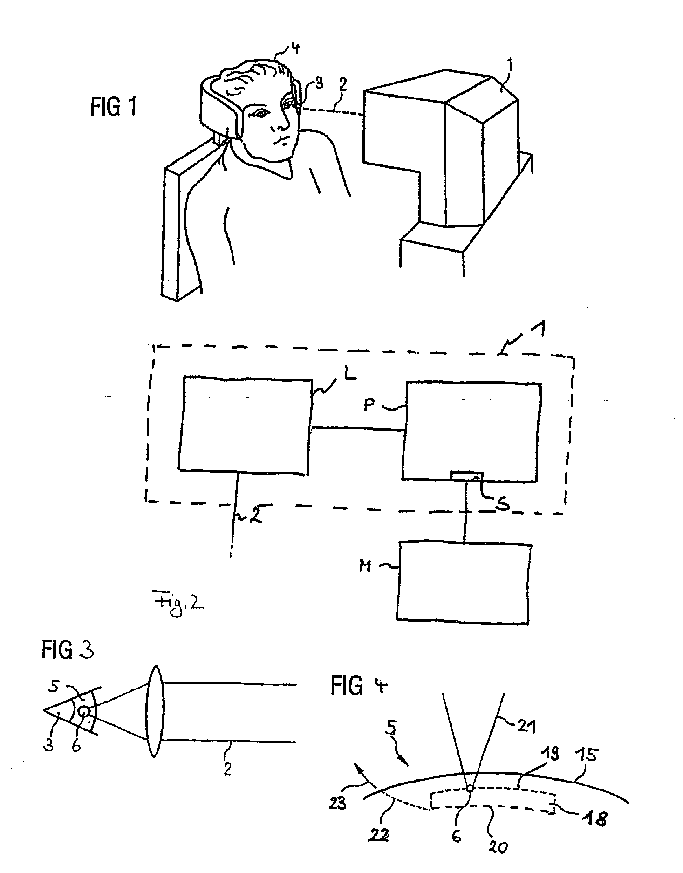

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 2 zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Meßeinrichtung M mit der Behandlungseinrichtung 1 hinsichtlich der Datenübertragung, die in einer Variante verwendet werden kann, hat den Vorteil, daß die Verwendung falscher Meß- und Fehlsichtigkeitsdaten mit größtmöglicher Sicherheit ausgeschlossen ist. Dies gilt insbesondere dann, wenn die Überführung des Patienten von der Meßeinrichtung M bzw. den Meßeinrichtungen zur Lasereinrichtung L mittels einer (in der Figur nicht dargestellten) Lagerungseinrichtung erfolgt, die mit der Meßeinrichtung M bzw. der Lasereinrichtung L so zusammenwirkt, daß die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Meßeinrichtung M zur Lasereinrichtung L kann dabei zugleich auch die Übertragung der Meß-und Fehlsichtigkeitsdaten an die Behandlungsvorrichtung 1 erfolgen.

Es ist vorzugsweise durch geeignete Mittel sichergestellt, daß die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 3 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus 6, der einen Spot überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot einen optischen Durchbruch in der Augenhornhaut 5 erzeugen, welcher wiederum eine in Figur 3 schematisch angedeutete Plasmablase initiiert. Dadurch wird dieses Laserpulses in der Hornhaut 5 Gewebe getrennt. Bei Entstehung einer Plasmablase umfaßt die Gewebeschichttrennung ein größeres Gebiet, als den Spot, welchen der Fokus 6 der Laserstrahlung 2 überdeckt, obwohl die Bedingungen zur Erzeugung des Durchbruches nur im Fokus 6 erreicht werden. Damit von jedem Laserpuls ein optischer Durchbruch von jedem Laserpuls erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, auch wenn in dieser Beschreibung gepulste Behandlungs-Laserstrahlung 2 geschildert wird. Beispielsweise kann ein Behandlungsgerät 1 verwendet werden, wie sie in der WO 2004/032810 A2 beschrieben ist. Eine Vielzahl von Laserpulsfoki bildet im Gewebe eine Schnittfläche aus, deren Form vom Muster abhängt, mit dem die Laserpulsfoki 6 im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche.

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

Die Laserstrahlung 2 wird, wie bereits erwähnt, in einem Fokus 6 in der Hornhaut 5 gebündelt, und die Lage des Fokus 6 in der Hornhaut wird verstellt, so daß zur Schnittflächenerzeugung an verschiedenen Stellen fokussiert Energie aus Laserstrahlungspulsen in das Gewebe der Hornhaut 3 eintragen wird. Die Laserstrahlung 2 wird von einem Laser als gepulste Strahlung bereitgestellt. Ein xy-Scanner, der in einer Variante durch zwei im wesentlichen orthogonal ablenkende Galvanometerspiegel realisiert ist, lenkt den vom Laser kommenden Laserstrahl zweidimensional ab, so daß nach dem xy-Scanner ein abgelenkter Laserstrahl vorliegt. Der xy-Scanner bewirkt somit eine Verstellung der Lage des Fokus 6 im wesentlichen senkrecht zur Haupteinfallsrichtung der Laserstrahlung 2 in die Hornhaut 5. Zur Verstellung der Tiefenlage ist neben dem xy-Scanner ein z-Scanner vorgesehen. Er sorgt dafür, daß die z-Position der Lage des Fokus 6, d.h. dessen Position auf der optischen Achse des Einfalls verändert wird. Der z-Scanner kann dem xy-Scanner nach- oder vorgeordnet sein. Die nachfolgend mit x, y, z bezeichneten Koordinaten beziehen sich also auf die Ablenkung der Lage des Fokus 6.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage des Fokus 6 in der Hornhaut 5 auch durch andere Koordinatensysteme erfolgen kann, insbesondere muß es sich nicht um ein rechtwinkliges Koordinatensystem handeln. Daß ein xy-Scanner um zueinander rechtwinklige Achsen ablenkt, ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 6 verwendet werden, wie dies nachfolgend auch noch erläutert wird. Beispiele für solche Koordinatensysteme sind insbesondere zylindrische Koordinaten.

Zur Steuerung der Lage des Fokus 6 werden der xy-Scanner sowie der z-Scanner, die gemeinsam ein konkretes Beispiel einer dreidimensionalen Fokusverstelleinrichtung realisieren, von einem im Behandlungsgerät (oder separat) vorgesehenen Steuergerät angesteuert. Gleiches gilt für den Laser. Das Steuergerät sorgt für einen geeignet synchronen Betrieb des Lasers sowie der dreidimensionalen Fokusverstelleinrichtung, exemplarisch realisiert durch den xy-Scanner sowie den z-Scanner, so daß die Lage des Fokus 6 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkretre Stellwerte für den Fokuslagenverstellmechanismus, z.B. für den xy-Scanner und den z-Scanner. Zur Vorbereitung des augenchirurgischen Verfahrens, also bevor das eigentliche Operationsverfahren ausgeführt werden kann, werden die Zielpunkte und auch deren Reihenfolge als Bahnkurve bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

Zuerst gilt es dazu das aus in der der Hornhaut 5 zu isolierende und später zu entfernende Volumen festzulegen. Dies ist im Stand der Technik bekannt.

Das Bearbeitungsgerät 1 erzeugt mittels der Laserstrahlung 2, die in das Auge fokussiert wird, eine gekrümmte Schnittfläche. Dabei wird der Fokus 6 entlang einer Bahn verstellt. Diese Schnittfläche, bei der sich beispielsweise um die Schnittfläche 19 oder 20 der Fig. 4 handeln kann, entsteht, indem der Fokus 6 entlang einer Bahnkurve verstellt wird. Um eine maximale Geschwindigkeit der Schnittflächenerzeugung zu erreichen, wird die Bahnkurve so gewählt, daß entlang derjenigen Verstellachse, die die langsamste ist, auch die geringste Verstellgeschwindigkeit erfordert ist. Die langsamste Verstellachse ist üblicherweise die z-Achse.

Die zu erzeugende Schnittfläche ist deshalb in eine oder mehrere Bahnkurven zu zerlegen, die sich bezogen auf die z-Achse an Höhenlinien der Schnittfläche orientieren. Die Erzeugung der Bahnkurve(n) ist der entscheidende Schritt zum Bereitstellen der Steuerdaten.

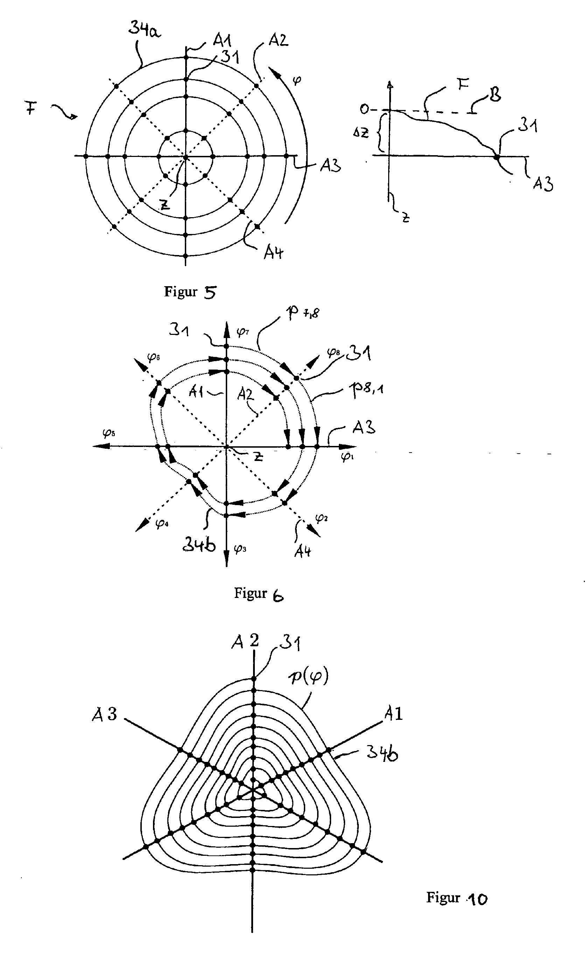

Fig. 5 zeigt exemplarisch eine Draufsicht auf die Schnittfläche F in Form eines Höhenlinienbildes 30. Dabei sei darauf hingewiesen, daß dieses Höhenlinienbild 30 lediglich zur Veranschaulichung der Zeichnung dient. Tatsächlich betrifft die hier beschriebene Ausführungsform Fälle, bei denen ein Höhenlinienbild gerade nicht verfügbar ist. Für die Schnittfläche F werden nun Achsen definiert, die nicht parallel zueinander liegen und in der beschriebenen Ausführungsform durch die Zylinderachse z laufen, die in der Darstellung der Fig. 5 senkrecht zur Zeichenebene liegt und mit dem Bezugszeichen "z" versehen ist. Im Beispiel der Fig. 5 sind vier Achsen A1, A2, A3 und A4 eingezeichnet.

Diese Achsen liegen alle in einer Bezugsebene und werden nun mit dieser in verschiedene Verschiebestellungen längs der z-Achse gebracht, und es werden jeweils Schnittpunkte 31 ermittelt, die die jeweilige Achse in der Verschiebestellung mit der Schnittfläche F hat. Der rechte Teil der Fig. 5 zeigt eine entsprechende Schnittdarstellung, die um 90° gegegenüber der Draufsicht des linken Teils der Fig. 5 gekippt ist. In dieser Darstellung ist die Achse A3 in eine Verschiebestellung Δz gegenüber dem Scheitelpunkt (eingetragen bei z-Koordinate 0), in dem die unverschobene Bezugsebene B liegt, verschoben. Es ergibt sich ein Schnittpunkt 31.

Die in den jeweiligen Verschiebestellungen für die Achsen erhaltenen Schnittpunkte 31 werden hinsichtlich ihrer Zylinderkoordinaten, d.h. hinsichtlich Radius r und Winkel ϕ sowie natürlich hinsichtlich der Verschiebestellung, d.h. der z-Koordinate, identifiziert. In der Draufsichtdarstellung des linken Teils der Fig. 5 ergeben sich dann eine Vielzahl von Schnittpunkten 31, welche die Achsen A1 bis A4 in den verschiedenen Verschiebestellungen, d.h. für verschiedene z-Koordinaten, haben.

Diese erhaltenen Schnittpunkte 31 werden nun durch eine Interpolation verbunden. Möchte man einen Satz geschlossener Bahnkurven 34a, werden jeweils die Schnittpunkte 31, welche sich in der selben Verschiebestellung, d.h. bei der selben z-Koordinate ergaben, verbunden. Bei der Verbindung werden winkelbenachbarte Schnittpunkte 31 durch eine Interpolation p miteinander verbunden. Die Interpolation erfolgt durch Variation des Radius r von der Winkelstellung des ersten Schnittpunktes 31 auf den Wert, den der Radius r bei der Winkelstellung des zweiten Schnittpunktes 31 hat. Die Interpolation wird stückweise ausgeführt und so gestaltet, daß die einzelnen Stücke glatt, d.h. stetig differenzierbar aneinanderschließen. Dies kann beispielsweise dadurch erreicht werden, daß als Randbedingung der Interpolation gefordert wird, daß die Steigung an jedem Schnittpunkt als Tangente zum jeweiligen Radius ausgebildet ist.

Möchte man statt einem Satz geschlossener Bahnkurven 34a eine Spirale 34b erhalten, die an Höhenlinien orientiert ist, werden ebenfalls winkelbenachbarte Schnittpunkte 31 verbunden, wobei jedoch zumindest innerhalb eines Umlaufs um 360° einmal die Verschiebestellung gewechselt wird, d.h. ein Schnittpunkt 31, der bei einer z-Koordinate ermittelt wurde, mit einem Schnittpunkt 31 verbunden wird, der in der nächst beabstandeten Verschiebestellung liegt, d.h. die nächstliegende z-Koordinate hat.

Dieses Vorgehen ist exemplarisch in Fig. 6 veranschaulicht. Auch hier sind wieder die vier Achsen A1, A2, A3 und A4 eingetragen, sowie die sich für die verschiedenen Verschiebestellungen ergebenden Schnittpunkte 31, wobei aufgrund der Projektion dies in Fig. 6 nicht zu erkennen ist. Jeder Achse A1 bis A4 sind Winkelwerte zugeordnet. Die Achse A1 hat die Winkelwerte ϕ₇ und ϕ₃, die Achse A2 die Winkelwerte ϕ₈ und ϕ₄, die Achse A3 die Winkelwerte ϕ₁ und ϕ₈ sowie die Achse A4 die Winkelwerte ϕ₂ und ϕ₆. Die zwei Winkelwerte jeder Achse unterscheiden sich um einen Winkel von 180°. Alternativ könnte man auch mit Halbachsen arbeiten. Dann hätte jede Achse einen eigenen Winkelwert und die Zahl der Achsen wäre verdoppelt.

Die stückweise Interpolation erfolgt im Beispiel der Fig. 6 durch Funktionen, die von dem Winkel einer Achse zum Winkel der nächsten laufen., beispielsweise vom ϕ₇ zum Winkel ϕ₈. Durch diese Interpolation mittels einer Interpolationsfunktion, in diesem Fall ϕ_{7,8}, wird ein Stück der Bahnkurve erhalten, das vom Schnittpunkt 31 der Achse A1 zum Schnittpunkt 31 der Achse A2 führt. Im einfachsten Fall bewirkt das Interpolationsstück p_{7,8} eine gleichmäßige Anpassung vom Radius, den der Schnittpunkt 31 der Achse A1 hat auf den Radius, der am Schnittpunkt 31 mit der Achse A2 vorliegt. Gleichartige Interpolationsstücke p schließen an, wie beispielsweise das Interpolationsstück p_{8,1}.

In der hier beschriebenen Ausführungsform liegen diese Interpolationsstücke alle noch bei derselben z-Koordinate, d.h. in derselben Verschiebestellung der Achsen. Ein Wechsel auf die nächste Verschiebestellung erfolgt im dargestellten Beispiel erst zwischen den Winkeln ϕ₃ und ϕ₄. Dieser Wechsel wiederholt sich beim nächsten Umlauf, so daß insgesamt eine Spirale 34b erhalten wird, die zwischen den Winkeln ϕ₄ und ϕ₃ Höhenlinien folgt, d.h. bei der selben z-Koordinate bleibt, und zwischen den Winkeln ϕ₃ und ϕ₄ in die nächste Verschiebestellung, d.h. die nächste z-Koordinate bzw. -Ebene übergeht.

Natürlich ist es alternativ auch möglich, die Übergang über einen größeren Winkelbereich zu verteilen, insbesondere gleichmäßig über 360° auszuführen. Dann liegen die Schnittpunkte 31 winkelbenachbarter Achsen, beispielsweise der Achsen A1 und A2, bei unterschiedlichen Verschiebestellungen. Das Vorgehen zur stückweisen Interpolation mittels der Interpolationsfunktion p ändert sich dadurch jedoch nicht, da diese Interpolationsfunktion lediglich eine Anpassung über den Winkel zwischen zwei aufeinanderfolgenden Winkelwerten vom Radius des ersten Winkelwertes auf den Radius des zweiten Winkelwertes bewirkt. Die z-Koordinate ist bei der stückweisen Interpolation nur dadurch zu berücksichtigen, daß mit einer durch den Abstand der Verschiebestellungen, der einem z-Vorschub entspricht, abhängig vom Winkel ϕ die z-Koordinate der Bahnfunktion entsprechend modifiziert wird. In die Ermittlung des radialen Überganges geht dies jedoch nicht ein. Mit anderen Worten, die Interpolationsfunktion läßt sich in einen (nur) vom Winkel abhängigen Radialanteil und einen ebenfalls (nur) vom Winkel abhängigen z-Anteil separieren. Diese Separation erlaubt es, die Interpolationsfunktion rechensparsam zu erzeugen.

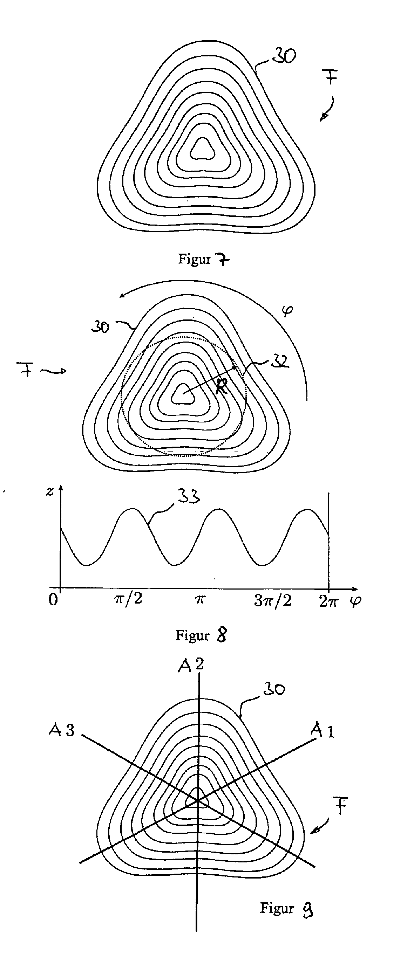

Für die Bestimmung der Schnittpunkte 31 ist die Lage der Achsen ausschlaggebend. Wie bereits im allgemeinen Teil der Beschreibung erwähnt, ist es zweckmäßig, die Winkelsymmetrie der Fläche F hierbei auszunutzen. Dies sei exemplarisch anhand einer dreizahlig symmetrischen Schnittfläche F erläutert, die in Fig. 7 wieder als Höhenlinienbild 30 dargestellt ist. Zur Festlegung der Lage der Achsen wird nun eine in ϕ periodische radiale Höhenfunktion z (r, ϕ) der Schnittfläche F auf einem Kreis mit Radius R ermittelt. Dieser Kreis 32 ist exemplarisch in Fig. 8 eingetragen. In Fig. 8 direkt darunter ist die Höhenfunktion 33 zu sehen. Im exemplarischen Fall erkennt man natürlich sofort, daß die höchste Periodizität pro Umlauf (2 π) drei ist. Es sind deshalb drei Achsen A1 bis A3 (bzw. sechs Halbachsen) ausreichend, wie Fig. 9 zeigt. Für komplexere Höhenfunktionen ist es zweckmäßig, diese in einer Funktionsreihe zu entwickeln, beispielsweise in eine Fourierreihe oder einer Zernike-Polynomreihe. Würde man die Höhenfunktion 33 in eine derartige Funktionenreihe entwickeln, sähe man, daß bei einer Entwicklung über die dritte Ordnung hinaus keine verbesserte Anpassung an die radiale Höhenfunktion erreicht wird.

Grundsätzlich kann man mit einer höheren Zahl von Achsen für manche Schnittflächen F eine bessere Anpassung erzielen. Gegenläufig dazu ist der mit der Zahl der Achsen steigende Rechenaufwand, da die Anzahl an Schnittpunkten 31 und die Anzahl an stückweisen Interpolationen entsprechend zunimmt. Es ist deshalb zweckmäßig, die Abweichung der Funktionsreihe von der Höhenfunktion mittels eines Schwellwertes (auch als Signifikanzschwelle bezeichnet) zu überprüfen. Die Entwicklung der Funktionsreihe wird dann abgebrochen, wenn die Abweichungen zwischen der Höhenfunktion und der Funktionsreihe unter dem Schwellwert bleibt.

In einem alternativen Vorgehen kann man auch die Extrema der Projektion der Schnittfläche F längs der z-Achse ermitteln (natürlich kann auch eine andere Achse verwendet werden). Die Zahl der Extrema gibt die Anzahl der erforderlichen Achsen an. Im Beispiel der Fig. 7 bzw. 9 erhielte man damit gleichermaßen drei Achsen (bzw. sechs Halbachsen).

Fig. 10 zeigt die spiralförmige Bahn 34b, welche sich an Höhenlinien orientiert und unter Auswertung der Schnittpunkte 31, welche die Achsen A1, A2 und A3 in der Schnittfläche 11 der Fig. 7 aufweisen, erhalten wurde. Für Fig. 10 gilt ansonsten das zu Fig. 6 gesagte analog.

## Patentansprüche

1. Verfahren zur Erzeugung von Steuerdaten, die ausgebildet sind zur Ansteuerung einer Laserstrahlung (2) abgebenden Lasereinrichtung (L) zur operativen Fehlsichtigkeitskorrektur eines Auges (3), wobei
- eine Schnittfläche (F) vorgegeben ist, die gekrümmt ist, einen Scheitelpunkt und einen Rand hat und zur Fehlsichtigkeitskorrektur im Auge (3) zu erzeugen ist,
- für die Steuerdaten eine oder mehrere Bahnkurve(n) (34a, 34b) definiert wird/werden, entlang der ein Fokus (6) der Laserstrahlung (2) zu verstellen ist,
- die Bahnkurve(n) (34a, 34b) so gewählt wird/werden, daß sie in der Schnittfläche (F) oder innerhalb eines Toleranzbereiches um die Schnittfläche (F) liegt/liegen,
**dadurch gekennzeichnet, daß** zur Wahl der Bahnkurve(n) (34a, 34b)
- eine Bezugsebene (B), welche in einem vorgegebenem Winkel, vorzugsweise senkrecht, zu einer Einfallsrichtung der Laserstrahlung (2) ist, festgelegt und für diese verschiedene Verschiebestellungen vom Scheitelpunkt zum Rand der Schnittfläche (F) festgelegt werden,
- für jede Verschiebestellung mehrere Achsen (A1, A2, A3, A4, ...) oder Halbachsen festgelegt werden, die in der Bezugsebene (B) liegen, zueinander nicht parallel sind und sich, gesehen in einer Projektion längs der Einfallsrichtung innerhalb des Randes der Schnittfläche, im Falle von Achsen (A1, A2, A3, A4) kreuzen und im Falle von Halbachsen mindestens einen gemeinsamen Punkt haben,
- für jede Verschiebestellung der Bezugsebene (B) Schnittpunkte (31) der Achsen (A1, A2, A3, A4) mit der Schnittfläche (F) ermittelt werden und
- die Bahnkurve(n) (34a, 34b) festgelegt wird/werden, indem die Schnittpunkte (31) durch eine Interpolation (p) zu in Projektion längs der Einfallsrichtung konzentrischen, geschlossenen Kurven (34a) oder in Form einer Spirale (34b) verbunden werden.

2. Verfahren nach Anspruch 1, **dadurch gekennzeichnet, daß** die Interpolation (p) stückweise zwischen jeweils zwei Schnittpunkten (31) erfolgt und ausgeführt wird, daß die Übergänge zwischen zwei aufeinanderfolgenden Interpolationstücken glatt sind.

3. Verfahren nach Anspruch 1 oder 2, **dadurch gekennzeichnet, daß** die Interpolation (p) in Zylinderkoordinaten (z, r, ϕ) erfolgt, wobei die Achsen (A1, A2, A3, A4, ...) so gewählt werden, daß eine Zylinderachse (Z) der Zylinderkoordinaten in jeder Verschiebestellung nahe eines Kreuzungspunktes der Achsen (A1, A2, A3, A4) liegt bzw. gemeinsamen Punktes der Halbachsen oder mit diesem zusammenfällt.

4. Verfahren nach einem der obigen Ansprüche, **dadurch gekennzeichnet, daß** die Achsen (A1, A2, A3, A4) oder Halbachsen winkelgleichverteilt sind.

5. Verfahren nach einem der obigen Ansprüche, **dadurch gekennzeichnet, daß** eine Winkelsymmetrie der Schnittfläche (F) beschreibende Symmetriezahl ermittelt und die Zahl der Achsen oder Halbachsen entsprechend der Symmetriezahl gewählt wird.

6. Verfahren nach Anspruch 5 in Verbindung mit Anspruch 3, **dadurch gekennzeichnet, daß** zur Bestimmung der Symmetriezahl zuerst eine Höhenfunktion (33) der Schnittfläche (F) auf einem Kreis (32) mit dem Radius R bestimmt wird und dann diese Höhenfunktion (33) in eine Funktionsreihe entwickelt wird, wobei die Symmetriezahl entsprechend einer maximalen Ordnung der Funktionsreihe festgelegt wird, die nötig ist, um eine Abweichung zwischen Entwicklung und Höhenfunktion (33) unter einen zuvor gewählten Schwellwert zu drücken.

7. Verfahren nach Anspruch 6, **dadurch gekennzeichnet, daß** die Bestimmung der Symmetriezahl für mehrere Radien R zwischen Null und dem maximalen Radius des Randes der Schnittfläche (F) wiederholt wird und die Zahl der Achsen oder Halbachsen entsprechend der größten erhaltenen Symmetriezahl gewählt wird.

8. Verfahren nach Anspruch 2 oder 3, **dadurch gekennzeichnet, daß** eine Höhenfunktion z(r, ϕ) der Schnittfläche auf einem Kreis (32) mit dem Radius R bestimmt wird, dann Winkellagen lokaler Extrema dieser Höhenfunktion (33) ermittelt werden und die Achsen oder Halbachsen auf die Winkellagen der lokalen Extrema gelegt werden.

9. Verfahren nach einem der obigen Ansprüche, **dadurch gekennzeichnet, daß** die Abstände Δz der Verschiebestellungen konstant sind oder einer vorgegebenen Funktion genügen.

10. Vorrichtung zur Erzeugung von Steuerdaten, die ausgebildet sind zur Ansteuerung einer Laserstrahlung abgebenden Lasereinrichtung (L) zur operativen Fehlsichtigkeitskorrektur eines Auges (3), wobei die Vorrichtung aufweist:
- eine Schnittstelle zum Empfang von Schnittstellendaten, die eine Schnittfläche (F) vorgeben, die gekrümmt ist, einen Scheitelpunkt und einen Rand hat und zur Fehlsichtigkeitskorrektur im Auge (3) zu erzeugen ist,
- eine Recheneinrichtung, die ausgebildet ist eine oder mehrere Bahnkurve(n) (34a, 34b) zu definieren, entlang der ein Fokus (6) der Laserstrahlung (2) zu verstellen ist, wobei die Recheneinrichtung die Bahnkurve(n) (34a, 34b) so wählt, daß sie in der Schnittfläche (F) oder innerhalb eines Toleranzbereiches um die Schnittfläche liegt/liegen,
**dadurch gekennzeichnet, daß** die Recheneinrichtung eingerichtet ist, ein Verfahren gemäß einem der obigen Ansprüche auszuführen.

11. Computerprogramm oder Computerprogrammprodukt, das einen Computer zur Ausführung eines der obigen Verfahren steuert.

## Claims

1. Method for generating control data that are intended to trigger a laser device (L) emitting laser radiation (2) for surgical correction of defective eyesight in an eye (3), wherein
- a sectional area (F) is predetermined, which is curved, has an apex and an edge, and is to be generated for correction of defective eyesight in the eye (3),
- for the control data one or more path curves (34a, 34b) is/are defined, along which a focal point (6) of the laser radiation (2) is to be adjusted,
- the path curve(s) (34a, 34b) is/are chosen so that it lies/they lie in the sectional area (F) or within a tolerance region around the sectional area (F),
**characterised in that** in order to choose the path curve(s) (34a, 34b)
- a reference plane (B), which lies at a predetermined angle, preferably perpendicular, to a direction of incidence of the laser radiation (2), is specified and for which various displacement settings from the apex to the edge of the sectional area (F) are specified,
- for each displacement setting a plurality of axes (A1, A2, A3, A4, ...) or semi-axes are specified, which lie in the reference plane (B), are not parallel to one another, and which viewed in a projection along the direction of incidence intersect within the edge of the sectional area in the case of axes (A1, A2, A3, A4) and in the case of semi-axes have at least one common point,
- for each displacement setting of the reference plane (B) intersection points (31) of the axes (A1, A2, A3, A4) with the sectional area (F) are determined, and
- the path curve(s) (34a, 34b) is/are specified, in which the intersection points (31) are connected by an interpolation (p) to closed (25) curves (34a) concentric in projection along the direction of incidence, or are connected in the form of a spiral (34b).

2. Method according to claim , **characterised in that** the interpolation (p) is performed piecewise between respectively two intersection points (31) and is executed so that the transitions between two successive interpolation pieces are smoother.

3. Method according to claim 1 or 2, **characterised in that** the interpolation (p) is performed in cylindrical coordinates (z, r, ϕ), wherein the axis (A1, A2, A3, A4, ...) are chosen so that a cylindrical axis (Z) of the cylindrical coordinates in each displacement setting lies close to a point of intersection of the axes (A1, A2, A3, A4) or a common point of the semi-axes or coincides with this.

4. Method according to one of the above claims, **characterised in that** the axes (A1, A2, A3, A4) or semi-axes are angularly equally distributed.

5. Method according to one of the above claims, **characterised in that** a symmetry number describing the angular symmetry of the sectional area (F) is determined and the number of axes or semi-axes is chosen corresponding to the symmetry number.

6. Method according to claim 5 in conjunction with claim 3, **characterised in that** in order to determine the symmetry number first of all a height function (33) of the sectional area (F) on a circle (32) of radius R is determined and this height function (33) is then expanded in a function series, wherein the symmetry number corresponding to a maximum order of the function series is specified, that is required in order to force a deviation between the expansion and height function (33) below a previously selected threshold value.

7. Method according to claim 6, **characterised in that** the determination of the symmetry number is repeated for several radii R between zero and the maximum radius of the edge of the sectional area (F) and the number of axes or semi-axes is selected corresponding to the largest obtained symmetry number.

8. Method according to claim 2 or 3, **characterised in that** a height function z(r, ϕ) of the sectional area on a circle (32) of radius R is determined, the angular positions of local extreme values of this height function (33) are determined, and the axes or semi-axes are placed on the angular positions of the local extreme values.

9. Method according to one of the above claims, **characterised in that** the interspacings Δz of the displacement settings are constant or satisfy a predetermined function.

10. Apparatus for generating control data that are intended to trigger a laser device (L) emitting laser radiation for surgical correction of defective eyesight in an eye (3), wherein the apparatus comprises:
- an interface for receiving interface data that predetermine a sectional area (F), which is curved, has an apex and an edge and is to be generated for correction of defective eyesight in the eye (3),
- a computing device that is designed to define one or more path curves (34a, 34b), along which a focal point (6) of the laser radiation (2) is to be adjusted, wherein the computing device selects the path curve(s) (34a, 34b) so that it lies/they lie in the sectional area (F) or within a tolerance region around the sectional area,
**characterised in that** the computing device is arranged to carry out a method according to one of the above claims.

11. Computer programs or computer program product that controls a computer for carrying out one of the above methods.

## Revendications

1. Procédé permettant de générer des données de commande, qui sont conçues pour activer un dispositif à laser (L) émettant un rayonnement laser (2) pour la correction chirurgicale de l'amétropie d'un oeil (3), dans lequel
- est prédéfinie une surface de coupe (F) qui est incurvée, possède un sommet et un bord et doit être générée pour la correction de l'amétropie dans l'oeil (3),
- une ou plusieurs orbites (34a, 34b) est/sont définie(s) pour les données de commande, le long de laquelle/desquelles orbite(s) doit être réglé un foyer (6) du rayonnement laser (2),
- la/les orbite(s) (34a, 34b) est/sont choisie(s) de telle sorte qu'elle(s) est/sont située(s) dans la surface de coupe (F) ou à l'intérieur d'une zone de tolérances autour de la surface de coupe (F),
**caractérisé en ce que** pour choisir la/les orbite (s) (34a, 34b)
- on définit un plan de référence (B) qui est situé en formant un angle prédéfini, de préférence un angle droit, avec une direction d'incidence du rayonnement laser (2), et différentes positions de déplacement du sommet vers le bord de la surface de coupe (F) sont définies pour ledit plan de référence,
- pour chaque position de déplacement on définit plusieurs axes (A1, A2, A3, A4, ...) ou demi-axes, qui sont situés dans le plan de référence (B), ne sont pas parallèles entre eux et, par référence à une projection le long de la direction d'incidence à l'intérieur du bord de la surface de coupe, se croisent dans le cas des axes (A1, A2, A3, A4) et ont au moins un point commun dans le cas des demi-axes,
- pour chaque position de déplacement du plan de référence (B), on détermine des points d'intersection (31) des axes (A1, A2, A3, A4) avec la surface de coupe (F), et
- la/les orbite(s) (34a, 34b) est/sont définie(s) en reliant les points d'intersection (31) par une interpolation (p) pour former des courbes (34a) fermées, concentriques dans la projection le long de la direction d'incidence ou pour former une spirale (34b).

2. Procédé selon la revendication 1, **caractérisé en ce que** l'interpolation (p) s'opère et est effectuée par tronçons entre respectivement deux points d'intersection (31), **en ce que** les transitions entre deux tronçons d'interpolation successifs sont lisses.

3. Procédé selon la revendication 1 ou 2, **caractérisé en ce que** l'interpolation (p) est effectuée dans des coordonnées cylindriques (z, r, ϕ), les axes (A1, A2, A3, A4, ...) étant choisis de telle sorte qu'un axe (Z) des coordonnées cylindriques se situe dans chaque position de déplacement à proximité d'un point d'intersection des axes (A1, A2, A3, A4) ou d'un point commun des demi-axes ou coïncident avec ceux-ci.

4. Procédé selon l'une quelconque des revendications précédentes, **caractérisé en ce que** les axes (A1, A2, A3, A4) ou demi-axes sont répartis selon des angles identiques.

5. Procédé selon l'une quelconque des revendications précédentes, **caractérisé en ce que** l'on détermine un nombre symétrique, définissant une symétrie angulaire de la surface de coupe (F), et le nombre d'axes et de demi-axes est choisi conformément au nombre symétrique.

6. Procédé selon la revendication 5 en association avec la revendication 3, **caractérisé en ce que** pour déterminer le nombre symétrique, on détermine d'abord une fonction de hauteur (33) de la surface de coupe (F) sur un cercle (32) avec le rayon R, et ensuite cette fonction de hauteur (33) est développée dans une série de fonctions, le nombre symétrique étant défini conformément à un ordre maximal de la série de fonctions qui est nécessaire pour qu'une divergence entre le développement et la fonction de hauteur (33) soit abaissée sous une valeur seuil choisie au préalable.

7. Procédé selon la revendication 6, **caractérisé en ce que** la détermination du nombre symétrique est répétée pour plusieurs rayons R entre zéro et le rayon maximum du bord de la surface de coupe (F), et le nombre d'axes ou de demi-axes est choisi conformément au plus grand nombre symétrique obtenu.

8. Procédé selon la revendication 2 ou 3, **caractérisé en ce que** l'on détermine une fonction de hauteur z(r, ϕ) de la surface de coupe sur un cercle (32) avec le rayon R, ensuite on calcule les positions angulaires des extrêmes locaux de cette fonction de hauteur (33), et les axes ou demi-axes sont posés sur les positions angulaires des extrêmes locaux.

9. Procédé selon l'une quelconque des revendications précédentes, **caractérisé en ce que** les distances Δz entre les positions de déplacement sont constantes ou satisfont à une fonction prédéfinie.

10. Dispositif permettant de générer des données de commande, qui sont conçues pour activer un dispositif à laser (L) émettant un rayonnement laser pour la correction chirurgicale de l'amétropie d'un oeil (3), ledit dispositif comportant :
- une interface pour recevoir des données d'interface qui prédéfinissent une surface de coupe (F) qui est incurvée, possède un sommet et un bord et doit être générée pour la correction de l'amétropie dans l'oeil (3),
- un centre de calcul, qui est configuré pour définir une ou plusieurs orbites (34a, 34b), le long de laquelle/desquelles doit être réglé un foyer (6) du rayonnement laser (2), ledit centre de calcul choisissant les orbite(s) (34a, 34b) de telle sorte qu'elle(s) est/sont située(s) dans la surface de coupe (F) ou à l'intérieur d'une zone de tolérances autour de la surface de coupe,
**caractérisé en ce que** le centre de calcul est configuré pour la mise en oeuvre d'un procédé selon l'une quelconque des revendications précédentes.

11. Programme informatique ou produit de programme informatique qui commande un ordinateur destiné à la mise en oeuvre du procédé ci-dessus.
